# EUROPEAN PATENT SPECIFICATION

(11) **EP 0 525 271 B1**
(45) Date of publication and mention of the grant of the patent: **04.09.1996**
(21) Application number: 91500082.2
(22) Date of filing: 29.07.1991
(51) Int. Cl.: C07C 219/06, D06M 13/463

(54) **Process for the preparation of quaternary compounds**
Verfahren zur Herstellung von quartären Verbindungen
Procédé pour la préparation de composés quaternaires

(43) Date of publication of application: 03.02.1993
(73) Proprietor: KAO CORPORATION, S.A., E-08210 Barbera del Valles, (Barcelona) (ES)
(72) Inventor: Vidal Boronat, Josep, E-08190 Sant Cugat del Valles, Barcelona (ES); Bermejo Oses, José, E-08840 Viladecans (ES)
(74) Representative: Duran Moya, Luis-Alfonso

(56) References cited:
- EP-A- 0 131 865
- EP-A- 0 252 441
- EP-A- 0 330 261
- CH-A- 210 485
- FR-M- 3 399

## Description

The present invention relates to a process for preparing etheramine esters and their quarternization products which can be used in the formulation of softeners and their typical textile treatment compositions and in hair conditioner compositions, which have excellent rewetting and biodegradability and good stability.

Quaternary nitrogen compounds have found commercial acceptance for fabric softening. These cationics have the most acceptable combination of properties including dispersability in water, resistance to yellowing, ability to be removed with subsequent washing.

Conventional softeners, salts of quaternary ammonium, are relatively inexpensive but offer some disadvantages: discolour fabrics, lessen rewetting of the fabrics, require various additives for successful domestic applications, and are severe eye and skin irritants.

To improve such disadvantages an important effort has been made in searching new cationic compounds with different chemical functional groups. Long chain alkyl groups compounds interrupted by carboxy groups (i.e., ester and amide) can be considered the substitutes of conventional softeners because not only do they yield good rewetting and softening properties to fibres but are also susceptible to be destroyed by living organisms.

U.S. Patent 3,915,867, Kang et al., October 28, 1975, relates the synthesis of a softener, ester ve, being characterized by whiteness retention and good rewetting properties.

U.S. Patent 0,293,955, Walley et al., December 7, 1988, relates the use of quaternary isopropyl ester ammonium compounds as fibre and fabric treatment compositions. The compositions described outstand for excellent storage stability and biodegradability.

U.S. Patent 0,295,739, Chang et al., December 21, 1988, encompasses a novel method for manufacturing aqueous biodegradable softening compositions. The softener ingredient used herein is an ester derivated from alkyl ethanol methyl amine.

U.S. Patent 4,439,331, Billenstein et al., Mar 27, 1984, describes a liquid textile softener composed of an aqueous solution of an ester diamine derivative. This textile softener contains nonionic contributing to make the softener easily dispersible in cold water.

U.S. Patent 4,720,383, Drach el al., Jan. 19, 1988 describes the synthesis of novel imidazolinium compounds and a method of using them as softening and conditioning agents for fibres, hair and skin.

U.S. Patent 4,540,521, Garst el al., Sep 10, 1985, describes a liquid quaternary ammonium composition obtained by quaternizing a mixture of an alkoxylated ether amine and a trialkanolamine. These quaternary compositions are useful as antistatic agents.

U.S. Patent 4,039,565, Throckmorton et al., Aug. 2, 1977, relates the synthesis of quaternized amidoamines and their use as fabric softeners.

EP-A-0 252 441 describes the preparation of Coconut (C₁₂-C₁₈)-alkyl-poly(6)oxyethyl-2-lauroyloxyethyl-2-hydroxyethyl-methyl-ammonium chloride.

The main specific problem in the use of these biodegradable cationic compounds is that this carboxyl group capable of inferring biodegradability to these softeners is also responsible for their hydrolysis.

The cationic compound described in this invention has the sufficient hydrolytic stability to be formulated as liquid compositions.

It is a novel feature of this invention to provide a process for preparing a cationic compound characterized by an ester group whose main chemical structure consists of two alkyl chains, and only a single ester group, instead of a mono- di- and trialkyl derivatives distribution.

The invention relates to a process for preparing a cationic compound with only a single ester group, providing better hydrolytic stability than conventional cationic compounds with two or more ester groups.

The etheramine ester and the quaternized etheramine ester prepared in the present invention exhibit rapid biodegradability relative to conventional fabric softening agents such as ditallow dimethyl ammonium chloride and ditallow imidazoline.

The use of alkoxylated alcohol as precursor in the synthesis of this cationic compound allows to obtain a product with lower melting temperature and consequently allows for liquid compositions. In this case the addition of ethoxylated alcohol to the composition for low viscosity at both normal and high temperatures and good product stability is not necessary.

The softener containing the compounds prepared according to this invention, in spite of not incorporating any rewetting agent, exhibits improved rewetting characteristics over conventional softening formulations which do incorporate them. In particular, there is provided a process for preparing an etheramine ester having the formula: 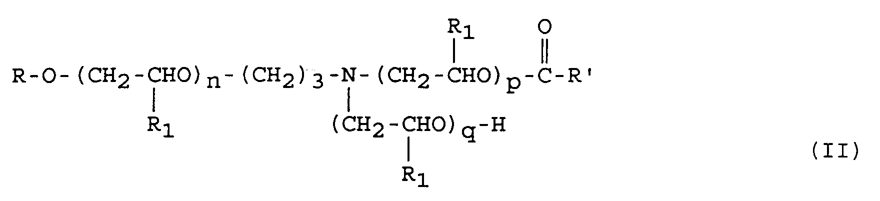
wherein R is a C₈-C₂₂ alkyl, alkenyl, aralkyl, alkylaryl or alkoxyalkyl, R' together with the carbonyl group to which it is attached is the residue of a C₈-C₂₂ fatty acid, each R₁ is independently a C₁-C₆ alkyl, alkoxy, hydroxyalkyl or hydrogen, n is 0-10, and p and q are 1-10, comprising the following reaction sequence:
(A) reacting acrylonitrile with R-O-(CH₂-CR₁HO)ₙ-H wherein R and R₁ are as defined above, to obtain the corresponding ethernitrile, at a temperature within the range of from 50 to 70 °C;
(B) hydrogenating the ethernitrile to obtain the corresponding etheramine, in the presence of ammonia at a temperature within the range of from 120 to 160 °C;
(C) reacting the etheramine with 1 to 2 moles of one or more optionally (R₁)-substituted ethylene oxides to obtain the corresponding alkoxylated etheramine; and
(D) reacting the alkoxylated etheramine with R'COOH or the corresponding methyl ester wherein R' is as defined above, to obtain the corresponding etheramine ester, at a temperature within the range of from 140 to 200 °C, preferably 140 to 170 °C, and the molar ratio of R'COOH or the corresponding methyl ester to the alkoxylated etheramine is from 1 to 2.
Further, there is provided a process for preparing a quaternary ammonium compound having the formula: 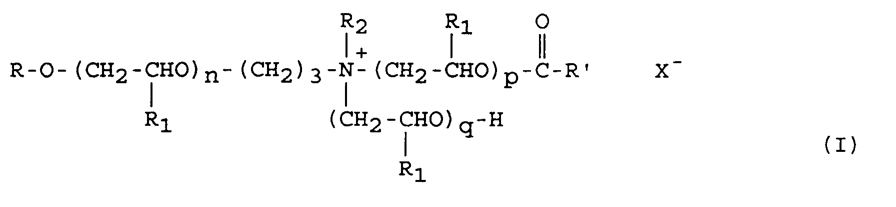
wherein R, R', R₁, n, p and q are as defined in claim 1, R₂ is a C₁-C₆ alkyl, hydroxyalkyl, benzyl or carboxymethyl group, X is a negatively charged ion, comprising the reaction sequence (A) to (D) of claim 1 and further
(E) quaternizing the etheramine ester with a quaternizing agent to obtain the quaternary ammonium compound, at a temperature of between 30 to 95 °C depending on the quaternizing agent.

The reaction sequences suitable for the preparation of the cationic compound are shown in scheme I:

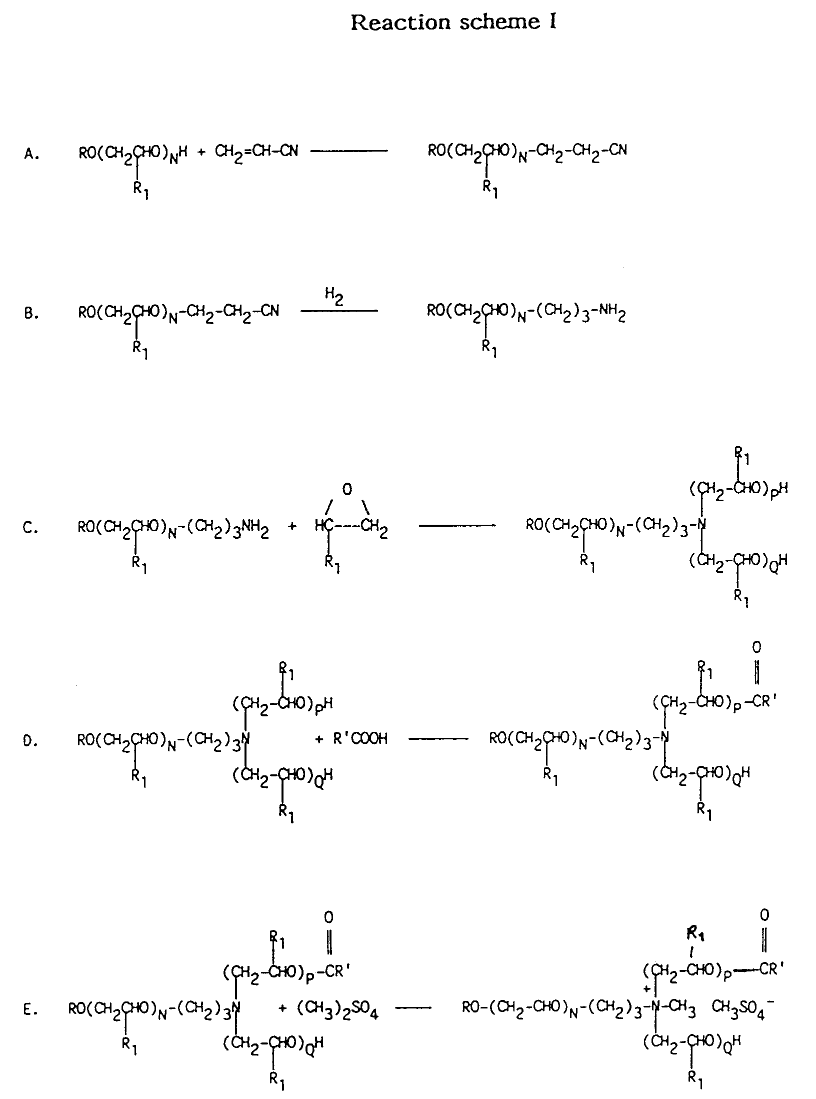

Reaction A - This step involves the reaction of one mole of acrylonitrile with about one mole of an alcohol containing from 8 to 22 carbon atoms or this alkoxylated alcohol (the degree of alkoxylation can go between 1 to 10 ) to form ethernitrile.It is well known that practically all primary and secondary alcohols react with acrylonitrile in the presence of alkaline catalysts. This reaction is clearly described in "Cyanoethylation", Organic Reactions, John Wiley and Sons, New York (1949).

Reaction B - The ethernitrile formed in the above reaction is then hydrogenated to obtain the corresponding etheramine. In the hydrogenation of nitriles to amines the presence of ammonia to suppress the formation of higher amines that reduce the content of primary amine in etheramine is very important.

Reaction C - The alkoxylated ether amines are obtained by reacting the ether amine with above defined ethylene oxide.

The degree of alkoxylation can be varied, if there is sufficient above defined ethylene oxide to react with both of the hydrogen atoms of the amine group, the resultant quaternary compound will be an ester amine, but if the molar ratio amine/oxide is higher than 0.5 the cationic compound will be a mixture of ester and amide derivatives. It is possible to prepare a polyalkoxylated ether amine by reacting the ether amine with more than two moles of alkyleneoxide.

The performance and physical properties (melting temperature, cloud point..) of these different kinds of compounds are slightly different.

Reaction D - This reaction provides for the conversion of the alkoxylated ether amine to the corresponding etheramine ester. It is carried out according to known methods from a C₈-C₂₂ fatty acid or methyl ester.

When the reagent is a fatty acid the reaction temperature is within the range of from 140 °C to 200 °C, preferably 140 °C to 170 °C. To accelerate the reaction, an acidic catalyst such as p-toluenesulfonic acid can be used. The reaction is controlled with respect to its completion by determining the acid number.

The molar ratio of fatty acid or methyl ester to alkoxylated ether amine is from 1 to 2. Depending on the molar ratio chosen, a mixture of fatty acid mono- and diester is obtained.

Reaction E - This reaction represents the conversion of ester ether amine to a quaternary compound. The ester amine is quaternized with a stoichiometric amount of di(lower alkyl)sulfate in accordance with conventional procedures.

Other known quaternizing agents such as methyl chloride or benzyl chloride may also be employed. The reaction takes place without the presence of a solvent or in a polar medium such as isopropanol at 30 °C - 95 °C.

### EXAMPLE 1

The synthesis of the biodegradable quaternary ammonium compound is accomplished by the following five-step process:

### Step A. Synthesis of Ethernitrile

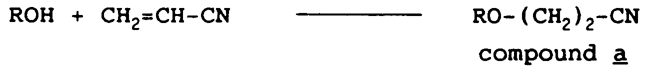

### PROCEDURE

80 g (0.3 moles) of tallow alcohol and 0.5 g of sodium methoxide as 30% solution in alcohol, are placed in a 250 ml flask equipped with mechanical stirrer, reflux condenser and thermometer. The temperature is raised to 70 °C, and 20 g (0.36 moles) of acrylonitrile are added dropwise with continued stirring at a temperature of about 60-70 °C for approximately 1 hour. The mixture is allowed to react an additional 2 hours.

The reaction mixture is then vacuum stripped at 80°C to remove volatiles and later filtered. The filtrate is an ethernitrile with a Gardner scale colour index lower than 1.

### ANALYSIS

IR 2210 , 1120 cm⁻¹

### Step B. Synthesis of Etheramine

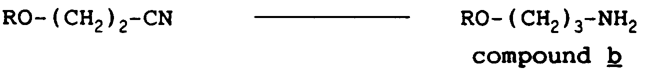

### PROCEDURE

100 g of said ethernitrile and 3 g of a commercial grade wet Raney nickel mud are charged to a stainless steel autoclave and purged three times with nitrogen, the autoclave is pressured to 6 Kg/cm² ammonia at 140 °C. Under continuous stirring the autoclave is held at 16 Kg/cm² H₂ for five hours at 140 °C. The reaction evolution is followed by titrating a sample for amine.

The autoclave is cooled and the product filtered, yielding the etheramine obtained about 95% primary amine and a colour lower than 1 Gd.

### ANALYSIS

IR 3200-3400, 1120 cm⁻¹

### Step C. Synthesis of Ethoxylated Etheramine

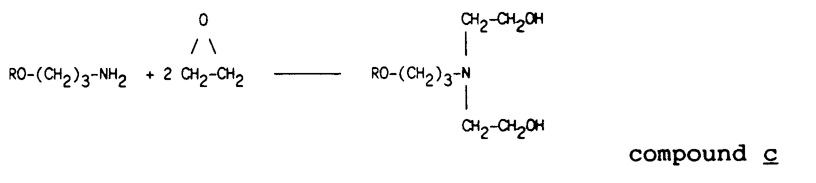

### PROCEDURE

100 g (0.31 moles) of etheramine are charged to a stainless steel reactor. The system is purged several times with nitrogen while heating to 140 °C. When the temperature reaches 140 °C, the reactor is pressurized to 2-3 Kg/cm² with ethylene oxide. The reaction temperature is kept at 130-140 °C and additional ethylene oxide is added until a total of 28 g (0.62 moles). After the final charge of ethylene oxide, the reaction mixture is allowed to react for about 1/2 hour, autoclave is cooled and discharged from reactor. The polyoxyethylene (2 OE) etheramine contains about 95% of tertiary amine, with a colour lower than 2 Gardner.

### ANALYSIS

IR 3200-3400, 1120, 1050 cm⁻¹

### Step D. Synthesis of Etheramine ester

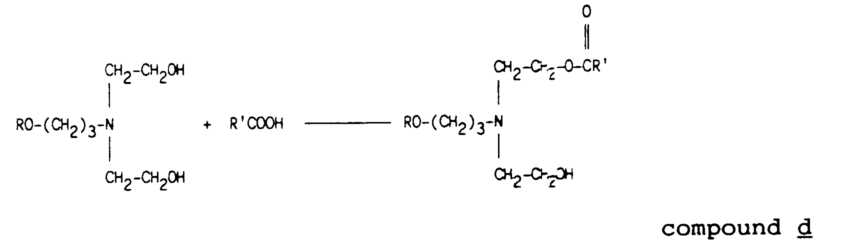

### PROCEDURE

100 g (0.25 moles) of ethoxylated etheramine are placed in a 250 ml flask in nitrogen atmosphere. The reaction mass is heated to 100 °C, and 82 g (0.3 moles) of tallow fatty acid are added dropwise under continuous stirring for about 1/2 hour at 100-110 °C. The reaction temperature is raised to 170 °C and kept for about 5 hours. Reaction evolution is followed by titrating acid value. The compound obtained contains about 98% ester etheramine, with a colour of 2-3 Gardner.

### ANALYSIS

IR 3200-3400, 1750, 1180, 1120, 1070 cm⁻¹

### Step E. Synthesis of Quaternary

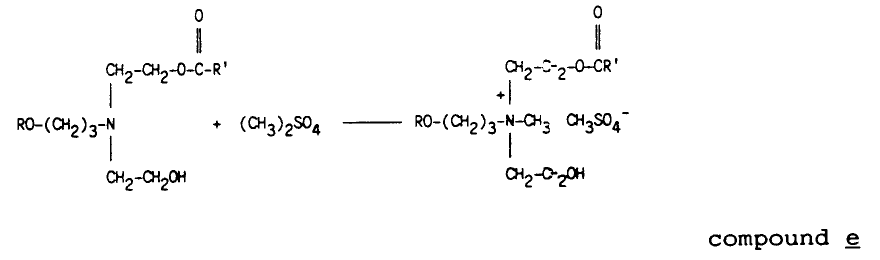

### PROCEDURE

100 g (0.15 moles) of ester etheramine and 21 g of isopropanol are placed in a 250 ml flask. The mixture is heated to 60 °C and 19 g (0.15 moles) of dimethyl sulphate are added dropwise under continuous stirring at about 60-65 °C for approximately 3 hours.
The quaternary compound is obtained with a colour of 2-3 Gardner.

### EXAMPLE 2

To demonstrate the versatility of the invention, the reaction sequences of example 1 were duplicated, except step C :

100 g (0.31 moles) of etheramine are charged to a stainless steel reactor. The system is purged several times with nitrogen while heating to 150 °C. When the temperature reaches 150 °C, the reactor is pressurized to 2-3 Kg/cm² with a mixture of ethylene oxide and propylene oxide at a ratio of 1/1. Reaction temperature is kept at 140-150 °C and additional oxides mixture is added to a total of 41 g. After the final charge of ethylene oxide the reaction mixture is allowed to react for about 1/2 hour, autoclave is cooled and discharged from reactor.

The poly(oxyethylene,oxypropylene) etheramine contains practically 100 % of tertiary amine.

### EXAMPLE 3

The starting alcohol agent of example 1 was replaced with an alcohol ethoxylated with 3 moles of ethylene oxide. The quaternary compound obtained had a melting point lower than the analogous cationic obtained in example 1.

## Claims

1. A process for preparing an etheramine ester having the formula: 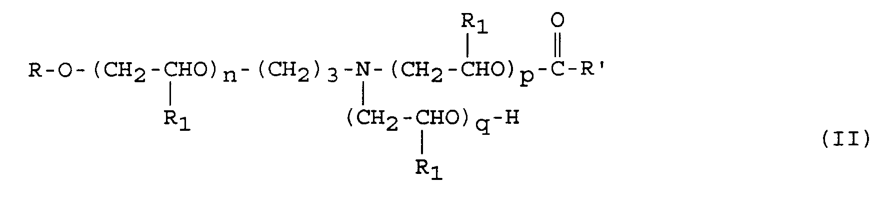 wherein R is a C₈-C₂₂ alkyl, alkenyl, aralkyl, alkylaryl or alkoxyalkyl, R' together with the carbonyl group to which it is attached is the residue of a C₈-C₂₂ fatty acid, each R₁ is independently a C₁-C₆ alkyl, alkoxy, hydroxyalkyl or hydrogen, n is 1-10, and p and q are 1-10, comprising the following reaction sequence:
(A) reacting acrylonitrile with R-O-(CH₂-CR₁HO)ₙ-H wherein R and R₁ are as defined above, to obtain the corresponding ethernitrile, at a temperature within the range of from 50 to 70 °C;
(B) hydrogenating the ethernitrile to obtain the corresponding etheramine, in the presence of ammonia at a temperature within the range of from 120 to 160 °C;
(C) reacting the etheramine with 1 to 2 moles of one or more optionally (R₁)-substituted ethylene oxides to obtain the corresponding alkoxylated etheramine; and
(D) reacting the alkoxylated etheramine with R'COOH or the corresponding methyl ester wherein R' is as defined above, to obtain the corresponding etheramine ester, at a temperature within the range of from 140 to 200 °C, preferably 140 to 170 °C, and the molar ratio of R'COOH or the corresponding methyl ester to the alkoxylated etheramine is from 1 to 2.

2. A process for preparing a quaternary ammonium compound having the formula: 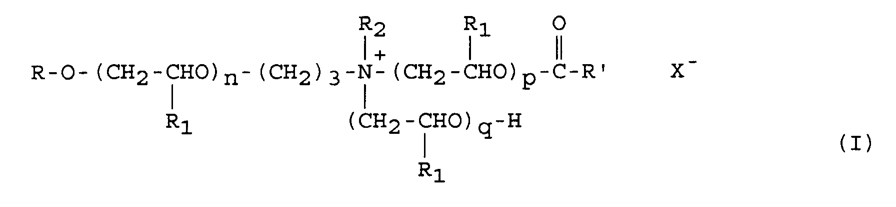 wherein R, R', R₁, n, p and q are as defined in claim 1, R₂ is a C₁-C₆ alkyl, hydroxyalkyl, benzyl or carboxymethyl group, X is a negatively charged ion, comprising the reaction sequence (A) to (D) of claim 1 and further
(E) quaternizing the etheramine ester with a quaternizing agent to obtain the quaternary ammonium compound, at a temperature of between 30 to 95 °C depending on the quaternizing agent.

## Patentansprüche

1. Verfahren zur Herstellung von Etheraminester der Formel: 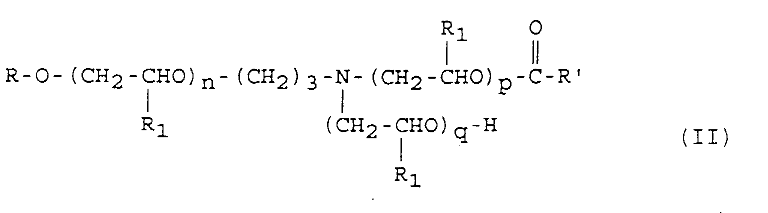 worin R eine C₈-C₂₂-Alkyl-, Alkenyl-, Aralkyl-, Alkylaryl- oder Alkoxyalkylgruppe ist, R' zusammen mit der Carbonylgruppe, an die es gebunden ist, den Rest einer C₈-C₂₂-Fettsäure darstellt, jedes R₁ unabhängig eine C₁-C₆-Alkyl-, Alkoxy-, Hydroxyalkylgruppe oder ein Wasserstoffatom ist, n 1 bis 10 ist und p und q 1 bis 10 sind, welches die nachstehende Reaktionsfolge umfaßt:
(A) Reaktion von Acrylonitril mit R-O-(CH₂-CR₁HO)ₙ-H, worin R und R₁ wie oben definiert sind, bei einer Temperatur im Bereich von 50 bis 70°C, wodurch das entsprechende Ethernitril erhalten wird;
(B) Hydrieren des Ethernitrils in Gegenwart von Ammoniak bei einer Temperatur im Bereich von 120 bis 160°C, wodurch das entsprechende Etheramin erhalten wird;
(C) Reaktion des Etheramins mit 1 bis 2 Molen eines oder mehrerer wahlfrei (R₁)-substituierter Ethylenoxide, wodurch das entsprechende alkyoxylierte Etheramin erhalten wird; und
(D) Reaktion des alkoxylierten Etheramins mit R'COOH oder dem entsprechenden Methylester, worin R' wie oben definiert ist, bei einer Temperatur im Bereich von 140 bis 200°C, vorzugsweise 140 bis 170°C und einem Molverhältnis von R'COOH oder dem entsprechenden Methylester zum alkoxylierten Etheramin von 1 bis 2, wodurch der entsprechende Etheraminester erhalten wird.

2. Verfahren zur Herstellung einer quaternären Ammoniumverbindung der Formel: 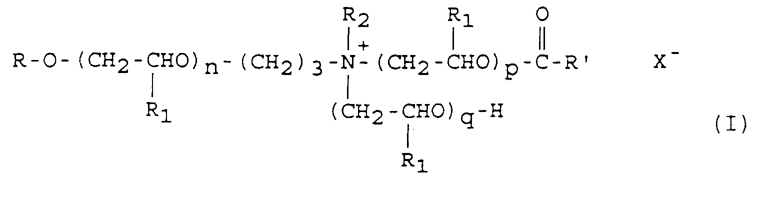 worin R, R', R₁, n, p und q wie in Anspruch 1 definiert sind, R₂ eine C₁-C₆-Alkyl-, Hydroxyalkyl-, Benzyl- oder Carboxymethylgruppe ist, X ein negativ geladenes Ion ist, das die Reaktionsfolge (A) bis (D) von Anspruch 1 und außerdem umfaßt:
(E) Quaternisierung des Etheraminesters mit einem Quaternisierungsmittel bei einer Temperatur, die in Abhängigkeit vom Quaternisierungsmittel zwischen 30 und 95°C liegt, wodurch die quaternäre Ammoniumverbindung erhalten wird.

## Revendications

1. Procédé qui consiste à préparer un ester d'éther-amine correspondant à la formule : 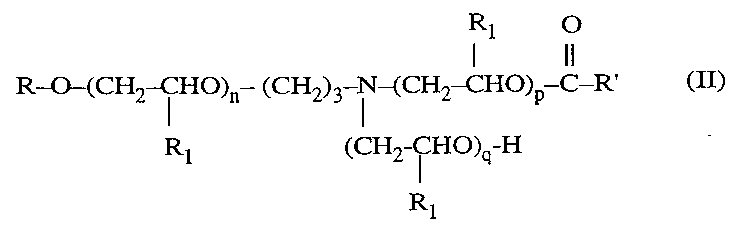 dans laquelle R représente un groupe alcényle, aralkyle, alkylaryle, alcoxyalkyle ou alkyle en C₈ - C₂₂, R' avec le groupe carbonyle auquel il est rattaché représente le résidu d'un acide gras en C₈ - C₂₂, chaque R₁ représente indépendamment un groupe alcoxy, hydroxyalkyle, alkyle en C₁ - C₆, ou un atome d'hydrogène, n est compris entre 1 et 10, et p et q sont compris entre 1 et 10, lequel procédé comprend la séquence réactionnelle suivante :
(A) le fait de faire réagir l'acrylonitrile et R-O-(CH₂-CR₁HO)ₙ-H dans laquelle R et R₁ sont tels que définis ci-dessus, afin d'obtenir l'éther-nitrile correspondant, à une température se situant dans l'intervalle allant de 50 à 70° C ;
(B) le fait d'hydrogéner l'éther-nitrile afin d'obtenir l'éther-amine correspondante en présence d'ammoniac à une température se situant dans l'intervalle allant de 120 à 160° C ;
(C) le fait de faire réagir l'éther-amine et 1 à 2 moles d'un ou plusieurs oxydes d'éthylène éventuellement substitués par des groupes R₁ afin d'obtenir l'éther-amine alcoxylée correspondante ; et
(D) le fait de faire réagir l'éther-amine alcoxylée et R'COOH ou l'ester méthylique correspondant, dans lequel R' est tel que défini ci-dessus, afin d'obtenir l'ester d'éther-amine correspondant, à une température se situant dans l'intervalle allant de 140 à 200° C, de préférence allant de 140 à 170° C, et le rapport molaire entre R'COOH ou l'ester méthylique correspondant et l'éther-amine alcoxylée vaut de 1 à 2.

2. Procédé qui consiste à préparer un composé de type ammonium quarternaire correspondant à la formule : 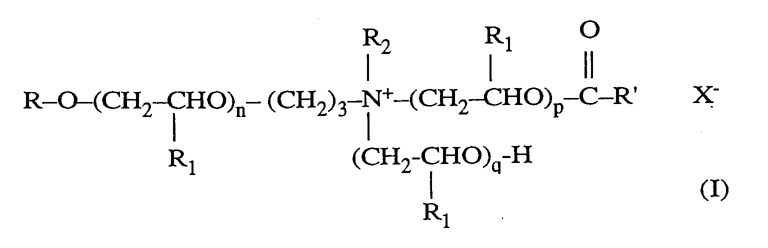 dans laquelle R, R', R₁, n, p et q sont tels que définis dans la revendication 1, R₂ représente un groupe benzyle, carboxyméthyle, hydroxyalkyle ou alkyle en C₁ - C₆, X représente un ion chargé négativement, lequel procédé comprend la séquence réactionnelle de (A) à (D) conforme à la revendication 1 et en outre
(E) le fait de transformer en dérivé quarternaire l'ester d'éther-amine avec un agent de quaternisation afin d'obtenir un composé de type ammonium quaternaire à une température comprise entre 30 et 95° C suivant l'agent de quaternisation.
